# FASCICULE DE BREVET EUROPEEN

(11) **EP 3 558 819 B1**
(45) Date de publication et mention de la délivrance du brevet: **05.02.2025**
(21) Numéro de dépôt: 17822324.4
(22) Date de dépôt: 22.12.2017
(51) Int. Cl.: G08B 21/12

(54) **SYSTÈME, PROCÉDÉ ET PRODUIT PROGRAMME D'ORDINATEUR DE DÉTERMINATION D'UNE NUISANCE GÉNÉRÉE PAR UNE INSTALLATION INDUSTRIELLE, INSTALLATION INDUSTRIELLE ÉQUIPÉE DU SYSTÈME**
SYSTEM, VERFAHREN UND COMPUTERPROGRAMMPRODUKT ZUR BESTIMMUNG EINER VON EINER INDUSTRIEANLAGE ERZEUGTEN BELÄSTIGUNG SOWIE INDUSTRIEANLAGE DAMIT
SYSTEM, METHOD AND COMPUTER PROGRAM PRODUCT FOR DETERMINING A NUISANCE GENERATED BY AN INDUSTRIAL INSTALLATION, AND INDUSTRIAL INSTALLATION EQUIPPED WITH THE SYSTEM

(30) Priorité: 22.12.2016 FR 1663178
(43) Date de publication de la demande: 30.10.2019
(73) Titulaire: Suez International, 92040 Paris la Défense Cedex (FR)
(72) Inventeur: FIEVEZ, Eric, 92150 Suresnes (FR); DELAHAYE, Mathieu, 78350 Jouy En Josas (FR); NASTASI, Valérie, 91140 Villejust (FR)
(74) Mandataire: Lavoix
(86) Numéro de dépôt international: PCT/EP2017/084507
(87) Numéro de publication internationale: WO 2018/115498

(56) Documents cités:
- EP-A2- 2 980 546
- EP-B1- 2 980 546
- CN-A- 103 823 028
- CN-A- 103 823 028
- CN-A- 105 775 134
- KR-A- 20100 111 263
- ANONYMOUS: "Comment mettre fin aux mauvaises odeurs - Environnement Magazine", 1 December 2010 (2010-12-01), XP055849451, Retrieved from the Internet <URL:https://www.environnement-magazine.fr/eau/article/2010/12/01/15685/comment-mettre-fin-aux-mauvaises-odeurs> [retrieved on 20211008]

## Description

### Domaine technique

L'invention se rapporte à un système et à un procédé de détermination d'un niveau de nuisance générée par une installation industrielle.

L'invention se rapporte aussi à un produit programme d'ordinateur pour mettre en œuvre le procédé selon l'invention et à une installation industrielle équipée du système selon l'invention.

### Etat de la technique

Les stations d'épuration ou de traitement de déchets ou d'autres installations industrielles d'autres domaines sont à l'origine de sources de nuisances, notamment olfactives ou sonores, impactant en particulier les riverains et sources d'émission de gaz à effet de serre, de gaz polluants et/ou dangereux (explosif).

Afin de disposer d'un ensemble de mesures suffisantes pour gérer de façon performante les émissions d'odeurs (ou sonores) d'une installation industrielle, les constructeurs ou les exploitants d'usines mettent en œuvre :
- soit des capteurs en poste fixe qui nécessitent d'être installés en nombre important afin de pouvoir collecter suffisamment de données, soit pour avoir suffisamment d'information en temps réel, soit pour pouvoir alimenter leur logiciel de prédiction de diffusion des odeurs. Néanmoins, ces capteurs sont installés près du sol (rarement à plus de 2 m) et en des endroits fixes. Le coût d'installation et de maintenance est multiplié par le nombre de capteurs ;
- soit des mesures ponctuelles faites par des capteurs portables. Cette méthode requiert la présence de personnel et n'est possible que ponctuellement, lors des essais de performance d'une installation neuve par exemple.

Un but de l'invention est de proposer un système de détermination d'un niveau de nuisance générée par une installation industrielle qui réduit le nombre de dispositifs de collecte nécessaire à la captation de ces données.

Un but de l'invention est de proposer un système de détermination d'un niveau de nuisance générée par une installation industrielle dont le coût d'installation et de maintenance est inférieur aux systèmes connus de l'art antérieur.

Le document "Comment mettre fin aux mauvaises odeurs - Environnement Magazine", 1 décembre 2010 (2010-12-01), XP055849451, divulgue les problématiques rencontrées par les installations industrielles de type stations d'épuration, des moyens existants pour caractériser et lutter contre des nuisances notamment oflactives.

Les documents EP2980546A2, CN103823028B, KR20100111263A et CN105775134A, divulguent des exemples d'utilisation de drones aériens dans des installations industrielles.

### Exposé de la technique

On atteint au moins un but de l'invention avec, selon un premier aspect de l'invention, un système de détermination d'un niveau de nuisance générée par une installation industrielle.

Le système selon l'invention comprend au moins un drone aérien, ledit drone aérien étant contrôlable pour se déplacer jusqu'à un point de mesure au-dessus, ou à proximité, de l'installation industrielle, et pour déterminer, au point de mesure, au moins un niveau d'une grandeur générant ladite nuisance, au moyen d'au moins un capteur équipant ledit drone aérien.

Par grandeur, dans le cadre de la présente invention, on entend grandeur physique et/ou grandeur chimique et/ou grandeur biologique.

On entend par à proximité de l'installation industrielle, dans un rayon d'environ 5km.

Le système selon l'invention comporte en outre une unité de traitement configurée pour déterminer le niveau de nuisance à partir du niveau de la grandeur captée.

La mise en œuvre d'un drone aérien permet de réduire le nombre de capteurs à installer.

En outre, le système permet de pouvoir mesurer les nuisances sur l'ensemble d'un site, voire en un endroit prédéterminé à l'avance.

Le drone aérien peut être ponctuellement déplacé en un point de mesure au niveau duquel l'installation d'un capteur fixe n'est pas possible, ou du moins au niveau duquel l'installation d'un capteur fixe engendrerait des coûts de maintenance élevés du fait, notamment, du remplacement nécessaire à court terme dudit capteur fixe. En outre, le drone aérien après avoir déterminé à un premier point de mesure un niveau de nuisance peut se déplacer vers un second point de mesure afin d'y déterminer un niveau de nuisance au niveau du second point de mesure. Ainsi, le système de détermination selon l'invention permet de minimiser l'investissement en nombre de capteurs, et ainsi le coût de maintenance des capteurs.

Par installation industrielle, l'invention vise, par exemple, des installations de traitement des eaux municipales et industrielles et des déchets, telles que des décharges - CET, des centres de tri, des incinérateurs, des usines de compostage, qui sont sources de pollutions olfactives et/ou sonores, de gaz polluants, sources d'effet de serre et/ou dangereux. Toute autre installation industrielle source de nuisances olfactives et/ou sonores est potentiellement concernée.

Par nuisance générée, on entend ainsi une pollution olfactive, voire également une pollution sonore, la présence de gaz polluants, une source d'effet de serre, ou encore la présence d'éléments dangereux.

Le système comporte au moins un drone aérien, c'est-à-dire un ou plusieurs drones aériens.

Le drone aérien peut être à énergie intrinsèque ou extrinsèque dans le cadre d'une alimentation extérieure.

Le drone aérien peut être contrôlé à distance, au moyen d'une liaison filaire ou sans fils.

L'unité de traitement peut équiper l'installation industrielle ou être située à distance de ladite installation industrielle ou bien être embarquée sur le drone aérien.

Le drone aérien peut comporter plusieurs capteurs, éventuellement de différents types, dont au moins un des capteurs est un capteur de concentration de gaz, le système selon l'invention permettant ainsi de déterminer un niveau de nuisance olfactive. Par exemple, un drone aérien peut comporter plusieurs capteurs de grandeurs différentes.

Les capteurs suivants sont envisagés, de façon non limitative : capteur d'ammoniac (NH3), capteur de mercaptans (RSH), capteur d'H2S, capteur de composés organiques volatils (COV), capteur de bruit (sonomètre), anémomètre, nez électroniques (les nez électroniques sont des systèmes qui détectent et mesurent des composés chimiques odorants et par un traitement des données permettent de quantifier le niveau d'odeur), multicapteurs permettant de déterminer les concentrations en polluants odorants et de calculer la concentration en unités d'odeur, d'autres types, comme NO2, SO2, CO, NOx.

Le système selon l'invention peut comporter des moyens de transmission, filaire ou sans fil, du niveau de la grandeur captée, à destination de l'unité de traitement.

Le système selon l'invention peut comprendre des moyens de régulation de la nuisance générée par l'installation industrielle, asservis en fonction du niveau de nuisance déterminé. Les moyens de régulation peuvent, par exemple, être une électrovanne dont la commande est asservie.

L'au moins un capteur peut être un anémomètre. La connaissance de la direction, du sens et de l'intensité du vent au point de mesure peut permettre d'affiner des prévisions relatives au transport du flux de la nuisance générée par l'installation industrielle.

L'au moins un capteur peut être un capteur sonore, typiquement un microphone ou sonomètre. Le système selon l'invention peut alors permettre de déterminer un niveau de nuisance sonore.

L'au moins un drone aérien est équipé d'une perche agencée pour disposer le capteur à distance dudit drone aérien. Cette perche peut alors permettre de minimiser l'influence de la présence du drone aérien sur le niveau déterminé de la grandeur. Cette perche peut être utile lorsqu'il est souhaité diminuer l'influence de la rotation d'hélices du drone aérien sur une mesure de vent réalisée par un anémomètre, sur une mesure sonore réalisée par un capteur sonore, sur une mesure de température réalisée par un capteur de température, thermomètre.

De préférence, le système selon l'invention comprend des moyens de communication sans fil qui équipent le drone aérien et l'installation industrielle et qui sont configurés pour établir une communication sans fil entre le drone aérien et l'installation industrielle.

Selon un mode de réalisation, le drone aérien comporte une unité de transmission configurée pour recevoir des commandes de déplacement. L'unité de transmission peut être configurée pour émettre le niveau de la grandeur captée à destination de l'unité de traitement.

Avantageusement, des commandes de déplacement à destination du drone aérien contrôlable et le niveau de la grandeur peuvent être transmis selon un protocole sans fil, par exemple selon le protocole WiFi.

Le système selon l'invention peut comprendre des relais de communications, filaires ou non, pour transmettre des commandes de déplacement à destination du drone aérien et/ou le niveau de la grandeur.

Selon un deuxième aspect de l'invention, il est proposé un procédé de détermination d'un niveau de nuisance générée par une installation industrielle, mis en œuvre dans un système de détermination selon le premier aspect de l'invention, ou l'un ou plusieurs de ses perfectionnements, comportant les étapes de :
- commande d'au moins un drone aérien équipé d'au moins un capteur d'une grandeur générant ladite au moins une nuisance, selon une trajectoire le conduisant à un point de mesure,
- détermination, par ledit capteur, d'un niveau de ladite grandeur au point de mesure,
- détermination, par une unité de traitement du système, dudit niveau de nuisance à partir de ladite valeur de ladite grandeur.

Plusieurs déterminations par l'au moins un capteur peuvent être réalisées au cours d'un même vol de l'au moins un drone aérien.

Plusieurs commandes peuvent être réalisées au cours d'un même vol de l'au moins un drone aérien.

Le point de mesure peut être situé en limite de propriété de l'installation industrielle. Par limite de propriété, la présente description vise tout point situé sur la frontière, tant horizontale que verticale, séparant la propriété comportant l'installation industrielle de l'extérieur de ladite propriété.

Selon une particularité du procédé selon l'invention, les étapes de commande et/ou de détermination peuvent être réalisées à intervalles réguliers.

De préférence, la trajectoire de l'au moins un drone aérien peut s'inscrire dans une zone de survol prédéterminée de l'installation industrielle. La zone de survol peut être déterminée à partir de réglementation s'appliquant au niveau de l'installation industrielle. La zone de survol peut être déterminée de sorte à limiter l'exposition du personnel de la zone industrielle à la circulation de l'au moins un drone aérien.

Avantageusement, le procédé selon l'invention comporte une étape de régulation de la nuisance générée par l'installation industrielle à partir du niveau de nuisance déterminé. Par exemple, une vanne peut être actionnée par un opérateur à partir de la connaissance du niveau de nuisance déterminé. Lorsqu'un moyen de régulation comporte un moyen de commande dudit moyen de régulation, par exemple lorsque c'est une électrovanne, l'étape de régulation peut comprendre un asservissement du moyen de régulation.

Le procédé selon l'invention peut comporter une étape de prédiction de l'évolution spatiale de la nuisance, et plus particulièrement de l'intensité de la nuisance, à partir d'un modèle recevant en entrée le niveau de nuisance déterminé.

L'étape de prédiction de l'évolution spatiale peut comporter un recalage du modèle par le niveau de nuisance déterminé. La détermination d'un niveau de nuisance peut ainsi permettre d'améliorer la prédiction de l'évolution spatiale de la nuisance.

Selon un autre aspect de l'invention, il est proposé un produit programme d'ordinateur, téléchargeable depuis un réseau de communication et/ou stocké sur un support lisible par ordinateur et/ou exécutable par un microprocesseur, caractérisé en ce qu'il comprend des instructions de code de programme pour la mise en œuvre d'un procédé de détermination d'un niveau de nuisance générée par une installation industrielle selon le premier aspect de l'invention, ou l'un ou plusieurs de ses perfectionnements.

Selon encore un autre aspect de l'invention, il est proposé une installation industrielle équipée d'un système de détermination selon le deuxième aspect de l'invention, ou l'un ou plusieurs de ses perfectionnements.

L'installation industrielle peut être équipée de l'unité de traitement configurée pour déterminer le niveau de nuisance à partir du niveau de la grandeur captée.

### Description des figures

D'autres particularités et avantages de l'invention apparaîtront à la lecture de la description détaillée de mise en œuvre et de mode de réalisation nullement limitatifs, au regard de figures annexées sur lesquelles :

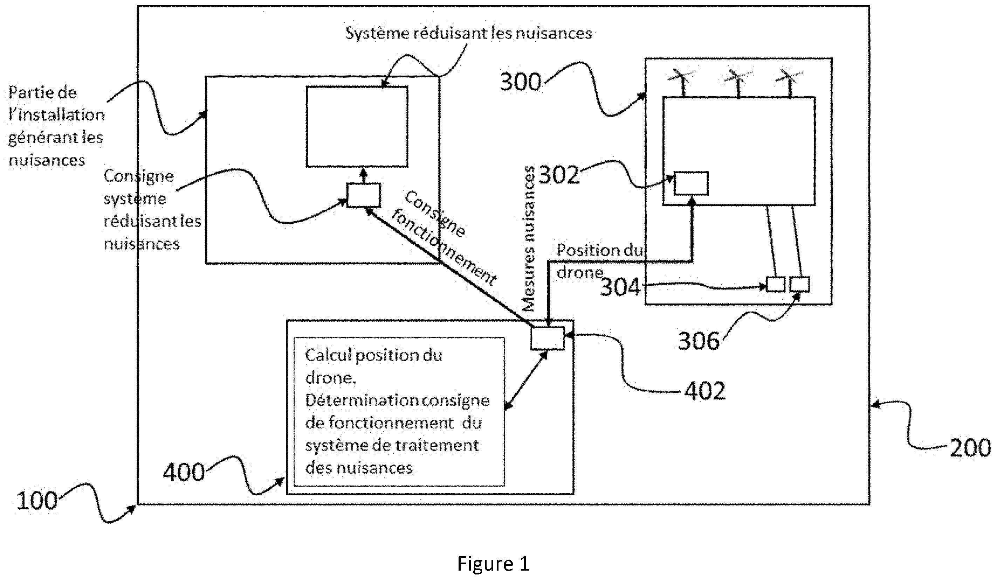

- la figure 1 illustre un premier mode de réalisation d'un système selon l'invention.

### Description de l'invention

Ce mode de réalisation n'étant nullement limitatif, on pourra notamment réaliser des variantes de l'invention ne comprenant qu'une sélection de caractéristiques décrites par la suite, telles que décrites ou généralisées, isolées des autres caractéristiques décrites, si cette sélection de caractéristiques est suffisante pour conférer un avantage technique ou pour différencier l'invention par rapport à l'état de la technique.

En référence à la figure 1, il est illustré un mode de réalisation d'un système 100 de détermination d'un niveau de nuisance générée par une installation industrielle 200. Dans l'exemple illustré, la nuisance est olfactive.

Le système 100 comprend :
- un drone aérien 300,
- une unité de traitement 400 équipant l'installation industrielle 200,
- une unité de transmission 402 équipant l'installation industrielle 200.

Le drone aérien 300 comporte une unité de transmission 302 configurée pour recevoir des commandes de déplacement, en provenance de l'unité de transmission 402. Le drone aérien 300 est contrôlable pour se déplacer jusqu'à un point de mesure au-dessus, ou à proximité, de l'installation industrielle 200.

Le drone aérien 300 comporte deux capteurs, 304 et 306, respectivement un anémomètre 304 et un capteur de concentration de gaz 306. Le drone aérien 300 est configuré pour déterminer, au point de mesure, un niveau de concentration de gaz générant la nuisance, olfactive dans l'exemple illustré.

L'unité de transmission 302 est en outre configurée pour émettre le niveau de la grandeur captée à destination de l'unité de traitement, via l'unité de transmission 302, ainsi que la direction, le sens et l'intensité du vent capté par l'anémomètre 304. Ces données sont reçues par l'unité de traitement 400 via l'unité de transmission 402.

Le transfert des données collectées en vol est réalisé, en tout ou partie au cours du vol, via une communication radio ou wifi ou tout autre mode de communication sans fil.

Le drone aérien 300 peut être commandé pour passer à différents endroits stratégiques de l'installation industrielle, par exemple :
- au niveau de sources principales d'émissions de nuisances (par exemple au niveau du stockage des boues sur une STEP, de l'entrée du local prétraitement, de l'épaississeur à boues, de la cheminée de sortie du système de désodorisation...),
- au niveau de la limite de propriété,
- au niveau de zones proches d'habitations,
- en altitude afin de mesurer le devenir des molécules olfactives une fois diffusées.

Le drone aérien 300 peut être commandé pour disposer de plusieurs chemins de parcours afin, par exemple, de passer par des endroits à fortes concentrations de gaz puis par des endroits à faible concentration en polluants.

Le drone aérien 300 peut être commandé pour se positionner en vol stationnaire à différents points du parcours pendant le temps nécessaire à une mesure stable et fiable des différents capteurs, pour une durée allant de la seconde à la minute par exemple.

Pour améliorer la qualité de la mesure, le drone aérien 300 est équipé d'une perche agencée pour disposer les capteurs 304 et 306 à distance dudit drone aérien.

Une fois son parcours effectué le drone aérien rejoint une base (non représentée) dans le but de recharger sa batterie (non représentée) afin de pouvoir réaliser le prochain parcours commandé,
L'unité de traitement 400 est configurée pour déterminer le niveau de nuisance à partir du niveau de ladite grandeur captée.

A partir de la grandeur captée, un exploitant peut prendre des actions préventives ou correctives afin de minimiser l'impact olfactif de l'installation industrielle. Ces actions peuvent par exemple être les suivantes :
- décaler des interventions de maintenance odorantes,
- optimiser le traitement des odeurs sur le site (par exemple en adaptant des taux de réactifs, modifiant des débits entrants dans la désodorisation),
- modifier des systèmes de ventilation,
- mettre en route un système de dispersion des odeurs,
- fermer les locaux,
- décaler l'intervention de prestataires,
- modifier le fonctionnement d'un processus de l'installation industrielle.

Lorsque l'installation industrielle est équipée d'un outil de contrôle des odeurs qui utilise les données reçues du drone aérien pour modéliser et simuler les concentrations en molécules odorantes dans le temps, ces actions peuvent être automatisées via l'outil de contrôle des odeurs. L'outil de prédiction de contrôle des odeurs peut comporter un recalage du modèle par le niveau de nuisance déterminé.

Le parcours du drone aérien peut être défini automatiquement à partir de la cartographie des odeurs déterminée par l'outil de contrôle.

Par exemple, lorsque la concentration en polluants à la sortie d'une cheminée de tours de désodorisation est supérieure à une consigne, le débit d'air entrant en désodorisation peut être automatiquement augmenté (ou le taux de traitement en réactifs). Ainsi, l'installation industrielle peut être équipée de moyens de régulation de la nuisance générée, asservis en fonction du niveau de nuisance déterminé.

Selon une utilisation complémentaire, le système selon l'invention peut être mis en œuvre dans le but de résoudre des problèmes d'exploitation de l'installation industrielle.

Le drone aérien peut par exemple être envoyé à des endroits critiques où l'on soupçonne un dysfonctionnement, par exemple sur un poste de relèvement ou une fuite sur un bâtiment.

Le drone aérien peut être commandé pour être positionné quelques minutes en sortie de la cheminée des tours de désodorisation afin de mesurer si celles-ci fonctionnent bien et identifier un possible dysfonctionnement. En fonction des données collectées, l'exploitant pourra prendre les mesures correctives nécessaires.

Cela est difficilement réalisable avec des capteurs fixes car la sortie de la cheminée est un endroit difficile d'accès. Leur installation les soumettrait à des conditions difficiles (humidité par exemple) qui rendent leur durée de vie et leur fiabilité de mesure faible.

Selon encore une autre utilisation, le système selon l'invention peut être mis en œuvre pour le contrôle de garanties de traitement lors de la construction d'installation industrielle. En effet, le constructeur d'une installation industrielle doit respecter des garanties en limite de propriété en termes de concentration d'odeur et de niveau de bruit. Il s'agit de différences de valeurs entre le niveau initial (sans l'installation industrielle) et le niveau constaté lors du fonctionnement de l'installation industrielle. Il est alors nécessaire de disposer d'une bonne connaissance du niveau initial pour bien distinguer les odeurs ou bruits provenant de la nouvelle installation industrielle de ceux provenant de l'environnement existant. L'invention permet de réaliser une carte initiale complète en survolant et en prenant un nombre important de mesures sur le site permettant une cartographie de la zone ce qui n'est pas réalisable avec des capteurs fixes. Ce même diagnostic est alors fait après la construction de l'installation industrielle.

Bien sûr, l'invention n'est pas limitée aux exemples qui viennent d'être décrits et de nombreux aménagements peuvent être apportés à ces exemples sans sortir du cadre de l'invention telle que définie par les revendications.

De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

## Revendications

1. Système (100) de détermination d'un niveau de nuisance olfactive générée par une installation industrielle (200), **caractérisé en ce qu'**il comprend au moins un drone aérien (300), ledit drone aérien étant contrôlable pour se déplacer jusqu'à un point de mesure au-dessus ou à proximité de ladite installation industrielle, et pour déterminer, au point de mesure, au moins un niveau d'une grandeur générant ladite nuisance, au moyen d'au moins un capteur (304, 306) équipant ledit drone aérien, ledit système comportant en outre une unité de traitement (400) configurée pour déterminer ledit niveau de nuisance à partir dudit niveau de ladite grandeur captée, dans lequel :
l'au moins un capteur (304, 306) est un capteur de concentration de gaz (306), et
le drone aérien (300) est équipé d'une perche agencée pour disposer le capteur (304, 306) à distance dudit drone aérien (300).

2. Système (100) selon la revendication précédente, comportant en outre des moyens de régulation de la nuisance générée par l'installation industrielle (200), asservis en fonction du niveau de nuisance déterminé.

3. Système (100) selon l'une quelconque des revendications précédentes, dans lequel ledit drone aérien (300) est équipé d'un anémomètre (304).

4. Système (100) selon l'une quelconque des revendications précédentes, dans lequel ledit drone aérien (300) est équipé d'un capteur sonore.

5. Système (100) selon l'une quelconque des revendications précédentes, comportant en outre des moyens de communication sans fil équipant le drone aérien (300) et l'installation industrielle (200) et configurés pour établir une communication sans fil entre le drone aérien (300) et l'installation industrielle (200).

6. Procédé de détermination d'un niveau de nuisance olfactive générée par une installation industrielle (200), mis en œuvre dans un système (100) de détermination selon l'une quelconque des revendications précédentes, comportant les étapes de :
commande d'au moins un drone aérien (300) équipé d'au moins un capteur (304, 306) d'une grandeur générant ladite au moins une nuisance, selon une trajectoire le conduisant à un point de mesure,
détermination, par ledit capteur (304, 306), d'un niveau de ladite grandeur au point de mesure,
détermination, par une unité de traitement (400) du système (100), dudit niveau de nuisance à partir de ladite valeur de ladite grandeur.

7. Procédé selon la revendication précédente, dans lequel le point de mesure est situé en limite de propriété de l'installation industrielle (200).

8. Procédé selon la revendication 6 ou 7, dans lequel l'étape de commande et de détermination sont réalisées à intervalles réguliers.

9. Procédé selon l'une quelconque des revendications 7 à 9, dans lequel la trajectoire s'inscrit dans une zone de survol prédéterminée de l'installation industrielle (200).

10. Procédé selon l'une quelconque des revendications 6 à 9, comportant en outre une étape de régulation de la nuisance générée par l'installation industrielle (200) à partir du niveau de nuisance déterminé.

11. Procédé selon l'une quelconque des revendications 6 à 10, comportant une étape de prédiction de l'évolution spatiale de la nuisance à partir d'un modèle recevant en entrée le niveau de nuisance déterminé.

12. Procédé selon la revendication 11, dans lequel l'étape de prédiction de l'évolution spatiale comporte un recalage du modèle par le niveau de nuisance déterminé.

13. Produit programme d'ordinateur téléchargeable depuis un réseau de communication et/ou stocké sur un support lisible par ordinateur et/ou exécutable par un microprocesseur, **caractérisé en ce qu'**il comprend des instructions de code de programme pour la mise en œuvre d'un procédé de détermination d'un niveau de nuisance générée par une installation industrielle (200) selon l'une quelconque des revendications 6 à 12.

14. Installation industrielle (200) équipée d'un système (100) de détermination selon l'une quelconque des revendications 1 à 5.

## Patentansprüche

1. System (100) zur Bestimmung eines Pegels von Geruchsbelästigung, die von einer Industrieanlage (200) erzeugt wird, **dadurch gekennzeichnet, dass** es mindestens eine Flugdrohne (300) umfasst, wobei die Flugdrohne steuerbar ist, um sich zu einem Messpunkt oberhalb oder in der Nähe der Industrieanlage zu bewegen, und um mittels mindestens eines Sensors (304, 306), mit dem die Flugdrohne ausgerüstet ist, an dem Messpunkt mindestens einen Pegel einer Größe zu bestimmen, die die Belästigung erzeugt, das System ferner umfassend eine Verarbeitungseinheit (400), die konfiguriert ist, um den Belästigungspegel anhand des Pegels der erfassten Größe zu bestimmen, wobei:
der mindestens eine Sensor (304, 306) ein Gaskonzentrationssensor (306) ist, und
die Flugdrohne (300) mit einer Stange ausgerüstet ist, die eingerichtet ist, um den Sensor (304, 306) in einem Abstand von der Flugdrohne (300) anzuordnen.

2. System (100) nach dem vorherigen Anspruch, ferner umfassend Regulierungseinrichtungen der von der Industrieanlage (200) erzeugten Belästigung, die abhängig von dem bestimmten Belästigungspegel geregelt werden.

3. System (100) nach einem der vorherigen Ansprüche, wobei die Flugdrohne (300) mit einem Anemometer (304) ausgerüstet ist.

4. System (100) nach einem der vorherigen Ansprüche, wobei die Flugdrohne (300) mit einem Schallsensor ausgerüstet ist.

5. System (100) nach einem der vorherigen Ansprüche, ferner umfassend drahtlose Kommunikationseinrichtungen, mit denen die Flugdrohne (300) und die Industrieanlage (200) ausgerüstet sind und die konfiguriert sind, um eine drahtlose Kommunikation zwischen der Flugdrohne (300) und der Industrieanlage (200) herstellen.

6. Verfahren zur Bestimmung eines Pegels von Geruchsbelästigung, die von einer Industrieanlage (200) erzeugt wird, das in einem Bestimmungssystem (100) nach einem der vorherigen Ansprüche durchgeführt wird, umfassend die folgenden Schritte:
Steuern mindestens einer Flugdrohne (300), die mit mindestens einem Sensor (304, 306) einer Größe ausgerüstet ist, die die mindestens eine Belästigung erzeugt, entlang einer Flugbahn, die sie zu einem Messpunkt führt,
Bestimmen, durch den Sensor (304, 306), eines Pegels der Größe an dem Messpunkt,
Bestimmen, durch eine Verarbeitungseinheit (400) des Systems (100), des Belästigungspegels anhand des Werts der Größe.

7. Verfahren nach dem vorherigen Anspruch, wobei sich der Messpunkt an der Grundstücksgrenze der Industrieanlage (200) befindet.

8. Verfahren nach Anspruch 6 oder 7, wobei der Steuerungs- und Bestimmungsschritt in regelmäßigen Abständen durchgeführt werden.

9. Verfahren nach einem der Ansprüche 7 bis 9, wobei die Flugbahn innerhalb eines vorbestimmten Überflugbereichs der Industrieanlage (200) liegt.

10. Verfahren nach einem der Ansprüche 6 bis 9, ferner umfassend einen Regulierungsschritt der Belästigung, die von der Industrieanlage (200) erzeugt wird, anhand des bestimmten Belästigungspegels.

11. Verfahren nach einem der Ansprüche 6 bis 10, umfassend einen Vorhersageschritt der räumlichen Entwicklung der Belästigung anhand eines Modells, das als Eingang den bestimmten Belästigungspegel erhält.

12. Verfahren nach Anspruch 11, wobei der Vorhersageschritt der räumlichen Entwicklung eine Neukalibrierung des Modells durch den bestimmten Belästigungspegel umfasst.

13. Computerprogrammprodukt, das aus einem Kommunikationsnetz herunterladbar ist und/oder auf einem computerlesbaren Medium gespeichert und/oder von einem Mikroprozessor ausführbar ist, **dadurch gekennzeichnet, dass** es Programmcodeanweisungen zur Durchführung eines Verfahrens zur Bestimmung eines Belästigungspegels, der von einer Industrieanlage (200) erzeugt wird, nach einem der Ansprüche 6 bis 12 umfasst.

14. Industrieanlage (200), die mit einem Bestimmungssystem (100) nach einem der Ansprüche 1 bis 5 ausgerüstet ist.

## Claims

1. A system for determining (100) an olfactory nuisance level generated by an industrial installation (200), **characterized in that** it comprises at least one aerial drone (300), said aerial drone being controllable for moving to a measuring point above or near said industrial installation, and for determining, at the measuring point, at least one level of a quantity generating said nuisance, by means of at least one sensor (304, 306) fitted to said aerial drone, said system further comprising a processing unit (400) configured for determining said level of nuisance based on said level of said sensed quantity, wherein:
the at least one sensor (304, 306) is a gas concentration sensor (306), and
the aerial drone (300) is fitted with a boom arranged for having the sensor (304, 306) at a distance from said aerial drone (300).

2. The system (100) according to the preceding claim, further comprising means for regulating the nuisance generated by the industrial installation (200), servo-controlled based on the determined level of nuisance.

3. The system (100) according to any one of the preceding claims, wherein said aerial drone (300) is fitted with an anemometer (304).

4. The system (100) according to any one of the preceding claims, wherein said aerial drone (300) is fitted with a sound sensor.

5. The system (100) according to any one of the preceding claims, further comprising wireless communication means fitted in the aerial drone (300) and the industrial installation (200) and configured for establishing wireless communication between the aerial drone (300) and the industrial installation (200).

6. A method for determining a level of olfactory nuisance generated by an industrial installation (200), implemented in a determination system (100) according to any one of the preceding claims, comprising the steps of:
controlling at least one aerial drone (300) fitted with at least one sensor (304, 306) of a quantity generating said at least one nuisance, along a trajectory leading it to a measuring point,
determining, by said sensor (304, 306), a level of said quantity at the measuring point,
determining, by a processing unit (400) of the system (100), said level of nuisance based on said value of said quantity.

7. The method according to the preceding claim, wherein the measuring point is located at the property boundary of the industrial installation (200).

8. The method according to claim 6 or 7, wherein the control and determination steps are performed at regular intervals.

9. The method according to any one of claims 7 to 9, wherein the trajectory falls within a predetermined overflight zone of the industrial installation (200).

10. The method according to any one of claims 6 to 9, further comprising a step of regulating the nuisance generated by the industrial installation (200) based on the determined level of nuisance.

11. The method according to any one of claims 6 to 10, comprising a step of predicting the spatial evolution of the nuisance based on a model receiving the determined level of nuisance as input.

12. The method according to claim 11, wherein the step of predicting the spatial evolution comprises a recalibration of the model by the determined level of nuisance.

13. A computer program product downloadable from a communication network and/or stored on a computer-readable medium and/or executable by a microprocessor, **characterized in that** it comprises program code instructions for implementing a method for determining a level of nuisance generated by an industrial installation (200) according to any one of claims 6 to 12.

14. An industrial installation (200) equipped with a determination system (100) according to any one of claims 1 to 5.
